(19) Europäisches Patentamt
European Patent Office
Office européen des brevets

(11) Publication number: 0 224 292 B1

# (12) EUROPEAN PATENT SPECIFICATION

(45) Date of publication of the patent specification: 11.04.90

(51) Int. Cl.⁴: C07C 265/12, C07C 263/14

(21) Application number: 86201944.5

(22) Date of filing: 06.11.86

## (54) Process for the preparation of organic isocyanates.

(30) Priority: 20.11.85 GB 8528532

(43) Date of publication of application: 03.06.87 Bulletin 87/23

(45) Publication of the grant of the patent: 11.04.90 Bulletin 90/15

(84) Designated Contracting States: BE CH DE ES FR GB IT LI NL

(56) References cited:
DE-A-1 815 517
DE-A-2 011 810
FR-A-2 175 086

(73) Proprietor: SHELL INTERNATIONALE RESEARCH MAATSCHAPPIJ B.V., Carel van Bylandtlaan 30, NL-2596 HR Den Haag(NL)

(72) Inventor: Drent, Eit, Badhuisweg 3, NL-1031 CM Amsterdam(NL)

(74) Representative: Aalbers, Onno et al, P.O. Box 302, NL-2501 CH The Hague(NL)

EP 0 224 292 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to a process for the preparation of organic isocyanates by reacting an aromatic nitro compound with carbon monoxide in the presence of a catalytic system based on palladium and/or a palladium compound and a group 5A ligand.

Organic isocyanates are valuable and versatile starting materials, for example for the production of polyurethanes and related compounds.

Numerous attempts have been made over the years to find alternative routes to the well-known preparation of isocyanates (or their precursors the carbamates), especially the most important ones such as toluenediisocyanate (TDI) and methylene-4,4′-diphenyldiisocyanate (MDI) via the so-called "phosgene route". Reference is made in this respect to "Recent Advances in Isocyanate Chemistry" by S. Ozaki-Chem. Rev., 72 (1972) 457-496.

According to US patent specification 3,914,268 organic isocyanates are prepared by contacting an aromatic nitro compound with carbon monoxide in the presence of a catalyst system comprising a platinum group metal compound, an aromatic nitrogenous heterocyclic compound and a component selected from water, hydrogen, hydrogen halides, boric acids, formic acid, oxalic acid and formaldehyde. A disadvantage of this known process is that carbon monoxide usually has to be applied at an extremely high partial pressure.

It has now surprisingly been found that organic isocyanates can be produced at a relatively low pressure, with a high reaction rate and with high selectivity when the process described in the beginning of this specification is carried out in the presence of a distinct type of ligand and of an acid. This favourable effect of the addition of an acid only occurs when the reaction is carried out with the distinct type of ligand used according to the invention, and not with ligands such as, for example, triphenylphosphine or pyridine.

Accordingly, the present invention provides a process for the preparation of organic isocyanates by reacting an aromatic nitro compound with carbon monoxide, which process is carried out in the presence of a catalytic system prepared by combining:-

a) palladium and/or a palladium compound,
b) a ligand having the general formula I

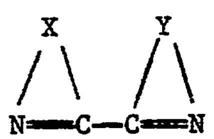

(I)

in which X and Y are the same or different bridging groups each of which has 3 or 4 atoms in the bridge, of which atoms at least two are carbon atoms and which groups X and Y may be bound to each other, and
c) one or more protonic acids, except hydrohalogenic acids.

The process according to the present invention is not only advantageous in that the aromatic nitro compound can be converted into the appropriate isocyanate with selectivities above 90%, but also because corrosion problems are of little significance. Furthermore, the recovery of palladium is not very cumbersome. Another important feature of the process of the invention is that it enables the preparation of isocyanates from aromatic nitro compounds and CO under moderate conditions in good yield and with a high reaction rate. In view of this the process is of great practical interest.

The bridging groups X and Y in formula I are connected with each other via the two carbon atoms shown in the formula. Apart from the connection as shown there may exist a second connection between the bridging groups, as is the case in 1,10-phenanthroline and derivatives thereof. Any atoms in the bridging groups X and Y other than carbon atoms are preferably nitrogen atoms. Furthermore, X and Y are preferably the same.

Examples of suitable bidentate ligands of formula I are 2,2′-bipyridyl and derivatives thereof, for example 4,4′-dimethyl-2,2′-bipyridyl, 4,4′-dichlorobipyridyl, 4,4′-dimethoxybipyridyl, 4,4′-dicarboxybipyridyl and 2,2′-biquinolyl.

Other examples of suitable bidentate ligands of formula I are 1,10-phenanthroline and derivatives thereof, for example 5-chlorophenanthroline, 4,7-diphenylphenanthroline, 4,7-dimethylphenanthroline, 2,9-dichlorophenanthroline, 1,10-phenanthroline-5-sulphonic acid and the 4,7-diphenyl-1,10-phenanthroline-disulphonic acids.

Further examples of suitable bidentate ligands of formula I are 2-(2-pyridyl)benzimidazole, 3-(2-pyridyl)-5,6-diphenyl-1,2,4-triazine and the monosodium salt of 3-(2-pyridyl)-5,6-diphenyl-1,2,4-triazine-p,p′-disulphonic acid.

The bidentate ligand preferably present in the catalytic system is 2,2′-bipyridyl or a derivative thereof or 1,10-phenanthroline or a derivative thereof. Particularly preferred are 2,2′-bipyridyl and 1,10-phenanthroline.

According to a preferred embodiment of the present invention use is made of one or more protonic acids having the general formula II

RQ (II)

in which R represents an optionally substituted aromatic group and Q represents an $SO_3H$, OH or C(O)OH group. The aromatic group represented by R may be, for example, a 1-naphthyl, 2-naphthyl, 1-,2- or 9-anthryl, 4-phenanthryl, or, what is preferred, an optionally substituted phenyl group. The aromatic group may be substituted with, for example, alkyl, cycloalkyl, phenyl, alkoxy, cycloalkoxy, phenoxy and, what is preferred, halogen atoms, i.e. iodine, bromine, chlorine and fluorine atoms. Very good results have been obtained with chlorinated aromatic groups. Preferred acids are 2,4,5-trichlorobenzenesulphonic acid, pentachlorophenol, 2,6-dichlorobenzoic acid and p-toluenesulphonic acid. Other examples of suitable acids of formula II are benzenesulphonic acid, 2,6-dichlorobenzenesulphonic acid, 2,6-dibromobenzenesulphonic acid, 2,6-difluorobenzenesulphonic acid, 2,4,6-trichlorobenzene sulphonic acid, 2,6-dichlorophenol, 2,6-difluorophenol, 2,4,6-tribromophenol, 2,4,6-trifluorophenol, 2,4,6-trichlorobenzoic acid, 2,4,6-tribromobenzoic acid and 2,4,6-trifluorobenzoic acid. The acid of the general formula II may be an ion exchange resin containing sulphonic acid groups, such as, for example, Amberlite 252H ("Amberlite" is a trade name), the group R being a polymeric hydrocarbon group, for example a polystyrene group substituted with sulphonic acid groups.

Use may be made of two or more different protonic acids of the general formula II, for example of an acid of formula II in which Q represents an $SO_3H$ group and one in which Q represents an OH group or a C(O)OH group, or of an acid of formula II in which Q represents an OH group and one in which Q represents a C(O)OH group.

Other examples of protonic acids that may be used in the process according to the present invention are those that can be formed, possibly in situ, by interaction of a Lewis acid, such as, for example, $BF_3$, $AsF_5$, $SbF_5$, $PF_5$, $TaF_5$ or $NbF_5$ with a Broensted acid such as, for example, a hydrohalogenic acid, in particular HF, fluorosulphonic acid, phoshoric acid or sulphuric acid. Specific examples of the last-named type of acids are $H_2SiF_6$, $HBF_4$, $HPF_6$ and $HSbF_6$. Examples of sulphonic acids that can be used are fluorosulphonic acid, trifluoromethylsulphonic acid, and chlorosulphonic acid. $HClO_4$ is another example of an acid that may be used.

Further examples of protonic acids are alkanoic acids, particularly those having not more than 12 carbon atoms per molecule, for example acetic acid, propionic acid, butyric acid, isobutyric acid, valeric acid, isovaleric acid, pivalic acid, heptanoic acid and lauric acid.

If use is made of n protonic acids, n being an integer, for example 2, not more than n-1 protonic acids may be present in the form of salts thereof with a transition metal, for example with copper, iron, cobalt, nickel, manganese, chromium, vanadium or titanium. For example, use may be made of a salt of a transition metal, particularly copper, of an acid of formula II in which R represents an optionally substituted phenyl group and Q an $SO_3H$ group, in combination with an acid of formula II in which R also represents an optionally substituted phenyl group and Q a C(O)OH group.

The process according to the present invention is carried out in the presence of palladium and or a palladium compound. Palladium can be used as such, deposited on an inert carrier such as carbon or alumina, or in the form of palladium compounds, especially palladium salts. Good results can also be obtained when a palladium compound is used which is substantially soluble in the prevailing reaction mixture.

Examples of convenient palladium salts include palladium chloride, palladium bromide, palladium iodide, sodium tetrachloropalladate, potassium tetrachloropalladate, potassium tetra-iodopalladate, palladium acetate, palladium propionate, palladium isobutyrate, palladium acetylacetonate and similar palladium compounds. Preference is given to the use of palladium salts of organic acids, in particular of an alkanoic acid having not more than 12 carbon atoms per molecule. Very good results have been obtained with palladium acetate.

As stated hereinbefore, the isocyanates are prepared from aromatic nitro compounds, i.e. compounds containing at least one aromatic group in which an $NO_2$ group is directly attached to a carbon atom forming part of an aromatic nucleus. Examples of aromatic nitro compounds include nitrobenzene, alkyl- and alkoxy-nitrobenzenes, aryl- and aryloxy-nitrobenzenes, dinitrobenzenes, alkyl- and alkoxy-, aryl- and aryloxy-dinitrobenzenes, such as 2,4-dinitrotoluene, 2,6-dinitrotoluene and 4,4′-dinitrodiphenylmethane, and polynitrobenzenes. Preferred aromatic nitro compounds are nitrobenzene, m-dinitrobenzene, 2-, 3- and 4-nitrotoluene, 2,4-dinitrotoluene, 2,6-dinitrotoluene and 4,4′-dinitrodiphenylmethane. Mixtures of two or more aromatic nitro compounds can be applied, e.g. a mixture of two dinitrotoluenes.

The amount of palladium or palladium compound to be used in the process according to the present invention is conveniently between 0.001 %w and 10 %w, in particular between 0.005 %w and 3 %w, calculated on the amount of nitro compound present in the reaction mixture. The use of rather small amounts of palladium compounds, e.g. less than 0.5 %w, calculated on nitro compound present in the reaction mixture, is preferred.

The ligands according to the general formula I may be used in such an amount that the ratio (palladium or palladium compound): ligand according to the general formula I is conveniently between 0.05 and 50, preferably between 0.1 and 20.

The amount of protonic acid present in the reaction mixture preferably lies between 0.01 and 150, more preferably between 0.1 and 100, and most preferably between 1 and 50 equivalents per gram atom of palladium. A molar ratio ligand of formula I to protonic acid in the range of from 0.2 to 5 is suitably used.

The process according to the present invention can be carried out conveniently at temperatures up to 300 °C. Preference is given to the use of temperatures in the range between 75 °C and 200 °C, in particular between 85 °C and 175 °C. The reaction is normally carried out at superatmospheric pressure; pressures of up to 500 bar can be applied. Preferably, the process is operated at a pressure in the range of from 10 to 150 bar. Good results can already be obtained using initial pressures between 30 and 80 bar.

The process according to the present invention is suitably carried out in the presence of an aprotic solvent. Examples of suitable solvents are hydrocarbons such as hexane, cyclohexane, octane, benzene, toluene, the threee xylenes, ethylbenzene and cumene; halogenated hydrocarbons such as chloroform, 1,2-dichloroethane, perfluoroalkanes, chlorobenzene and the three dichlorobenzenes; ethers such as tetrahydrofuran, diethyl ether, 3,6-dioxaoctane, methyl tert.-butyl ether, dioxane, anisole, 2,5,8-trioxanonane (also referred to as "diglyme") diphenyl ether and diisopropyl ether; N,N-dialkyl-substituted amides, such as N,N-dimethylformamide and N-methylpyrrolidone; sulphones such as diisopropyl sulphone and tetrahydrothiophene 1,1-dioxide (also referred to as "sulpholane"); esters such as methyl benzoate and ethyl acetate.

The process according to the present invention can be carried out batchwise, semi-continuously or continuously. The reaction time is, of course, related to the temperature and pressure adopted. In general, reaction times between 1 and 20 hours appear to be adequate.

The following examples further illustrate the invention. The experiments were carried out in a 300 ml magnetically stirred Hastelloy C autoclave ("Hastelloy" is a trade mark). The selectivity to a certain compound, expressed in a percentage, is defined herein as 100 × a/b, in which "a" is the amount of starting compound that has been converted into that certain compound and "b" is the total amount of starting compound that has been converted.

Examples 1-7

Example 1 was carried out as follows. The autoclave was charged with nitrobenzene (7.5 ml), toluene (50 ml), palladium(II) acetate (0.8 mmol), 2,2'-bipyridyl (10 mmol), p-toluenesulphonic acid (1 mmol) and pentachlorophenol (5 mmol). Then, consecutively, the autoclave was pressurized with carbon monoxide until an initial pressure of 70 bar was reached, the temperature was raised to 150 °C, the reaction mixture was kept at this temperature for 5h, the reaction mixture was allowed to cool to ambient temperature and was analysed using gas-liquid chromatography. The conversion of nitrobenzene was 90% with a selectivity to phenyl isocyanate of 80%. Table 1 hereinafter shows the relevant data of this example; p-toluenesulphonic acid is abbreviated to "pTsA" in this table.

Examples 2-7 were carried out in the same manner as Example 1, but with the differences as stated in Table 1; the results of Examples 4-7 were measured after 3 h. This table also presents the results.

Table 1

| Example | 2,2'-bipyridyl mmol | Acids (mmol) | | Solvent | Conversion of nitrobenzene, % | Selectivity to phenyl isocyanate % |
|---|---|---|---|---|---|---|
| 1 | 10 | pTsA (1) | pentachlorophenol (5) | toluene | 90 | 80 |
| 2 | ditto | | pentachlorophenol (5) | ditto | 20 | 75 |
| 3 | ditto | pTsA (1) | | ditto | 60 | 60 |
| 4 | ditto | pTsA (1) | 2,6-dichlorobenzoic acid (10) | p-xylene | 67 | 73 |
| 5 | 20 | 2,4,5-trichloro-benzenesulphonic acid (1) | 2,6-dichlorobenzoic acid (10) | ditto | 62 | 83 |
| 6 | ditto | ditto (1) | ditto (20) | ditto | 50 | 92 |
| 7 | 10 | pTsA (1) | pentachlorophenol (10) | diglyme | 60 | 85 |

Example 8

The autoclave was charged with nitrobenzene (7.5 ml), diglyme (50 ml), palladium (II) acetate (0.8 mmol), 2,2'-bipyridyl (20 mmol), p-toluenesulphonic acid (1 mmol) and acetic acid (20 mmol). Then, consecutively, the autoclave was pressurized with carbon monoxide (initial pressure 60 bar), the temperature was raised to 150 °C, the reaction mixture was kept at this temperature for 1.5 h and the reaction mixture was allowed to cool to ambient temperature. The conversion of nitrobenzene was 80%, with a selectivity to phenyl isocyanate of 61%.

Example 9

The autoclave was charged with nitrobenzene (15 ml), diglyme (42.5 ml), palladium (II) acetate (0.8 mmol), 2,2'-bipyridyl (20 mmol), cupric tosylate (1 mmol) and 2,6-dichlorobenzoic acid (10 mmol). Then, consecutively, the autoclave was pressurized with carbon monoxide (inital pressure 70 bar), the temperature was raised to 150 °C, the reaction mixture was kept at this temperature for 5 h and the reaction mixture was allowed to cool to ambient temperature. The conversion of nitrobenzene was 40%, with a selectivity to phenyl isocyanate of 85%.

Example 10

The autoclave was charged with nitrobenzene (15 ml), p-xylene (50 ml), palladium(II) acetate (0.8 mmol), 1,10-phenanthroline (10 mmol), 2,4,5-trichlorosulphonic acid (1 mmol) and 2,6-dichlorobenzoic acid (10 mmol). Then, consecutively, the autoclave was pressurized with carbon monoxide (initial pressure 70 bar), the temperature was raised to 150 °C, the reaction mixture was kept at this temperature for 3 h and the reaction mixture was allowed to cool to ambient temperature. The conversion of nitrobenzene was 46%, with a selectivity to phenyl isocyanate of 60%.

Comparative Experiment

The autoclave was charged with nitrobenzene (7.5 ml), diglyme (50 ml), palladium(II) acetate (0.2 mmol), pyridine (20 mmol), p-toluenesulphonic acid (1 mmol) and acetic acid (20 mmol). Then, consecutively, the autoclave was pressurized with carbon monoxide (initial pressure 60 bar), the temperature was raised to 150 °C, the reaction mixture was kept at this temperature for 5 h and the reaction mixture was allowed to cool to ambient temperature. Phenyl isocyanate could not be detected in the reaction mixture.

## Claims

1. Process for the preparation of organic isocyanates by reacting an aromatic nitro compound with carbon monoxide, which process is carried out in the presence of a catalytic system prepared by combining:-

a) palladium and/or a palladium compound,

b) a ligand having the general formula I

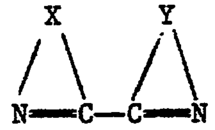

(I)

in which X and Y are the same or different bridging groups each of which has 3 or 4 atoms in the bridge, of which atoms at least two are carbon atoms and which groups X and Y may be bound to each other, and

c) one or more protonic acids, except hydrohalogenic acids.

2. Process as claimed in claim 1, in which X and Y are also bound to each other by means of a connection other than that formed by the carbon atoms shown in formula I.

3. Process as claimed in claim 2, in which the ligand is 1,10-phenanthroline or a derivative thereof.

4. Process as claimed in claim 2, in which the ligand is 2,2'-bipyridyl or a derivative thereof.

5. Process as claimed in any one of the preceding claims, in which use is made of one or more protonic acids having the general formula II

RQ (II)

in which R represents an optionally substituted aromatic group and Q represents an $SO_3H$, OH or C(O)OH group.

6. Process as claimed in claim 5, in which R represents an optionally substituted phenyl group.

7. Process as claimed in claim 5 or 6, in which the aromatic group is halogenated.
8. Process as claimed in claim 7, in which the aromatic group is chlorinated.
9. Process as claimed in claim 8, in which use is made of 2,4,5-trichlorobenzenesulphonic acid.
10. Process as claimed in claim 8, in which use is made of pentachlorophenol.
11. Process as claimed in claim 8, in which use is made of 2,6-dichlorobenzoic acid.
12. Process as claimed in claim 6, in which use is made of p-toluenesulphonic acid.
13. Process as claimed in any one of the preceding claims, in which the palladium compound is a palladium salt of an organic acid.
14. Process as claimed in any one of the preceding claims, which is carried out at a temperature in the range of from 75 °C to 200 °C.
15. Process as claimed in any one of the preceding claims, which is carried out at a pressure in the range of from 10 to 150 bar.

**Patentansprüche**

1. Verfahren zur Herstellung von organischen Isocyanaten durch Umsetzung einer aromatischen Nitroverbindung mit Kohlenmonoxid, wobei das Verfahren durchgeführt wird in Gegenwart eines katalytischen Systems, das hergestellt worden ist durch Zusammenbringen von
a) Palladium und/oder einer Palladiumverbindung,
b) einem Liganden der allgemeinen Formel I

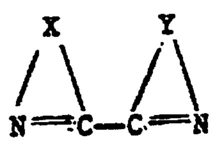

(I)

in der X und Y gleiche oder unterschiedliche Brückengruppen darstellen, von denen jede 3 oder 4 Atome in der Brücke aufweist, wobei von den Atomen mindestens zwei Kohlenstoffatome sind, und wobei die Gruppen X und Y aneinander gebunden sein können, und
c) einer oder mehrerer Protonensäuren, mit Ausnahme von Halogenwasserstoffsäuren.
2. Verfahren nach Anspruch 1, wobei X und Y auch aneinander gebunden sind durch eine andere Bindung als diejenige, die durch die in Formel I angegebenen Kohlenstoffatome gebildet wird.
3. Verfahren nach Anspruch 2, wobei der Ligand 1,10-Phenanthrolin oder ein Derivat davon ist.
4. Verfahren nach Anspruch 2, wobei der Ligand 2,2'-Bipyridyl oder ein Derivat davon ist.
5. Verfahren nach einem der vorangehenden Ansprüche, wobei eine oder mehrere Protonensäuren angewandt werden, der allgemeinen Formel II

RQ (II),

in der R eine gegebenenfalls substituierte aromatische Gruppe und Q eine $SO_3H$-, OH- oder C(O)OH-Gruppe bedeutet.
6. Verfahren nach Anspruch 5, wobei R eine gegebenenfalls substituierte Phenylgruppe bedeutet.
7. Verfahren nach Anspruch 5 oder 6, wobei die aromatische Gruppe halogeniert ist.
8. Verfahren nach Anspruch 9, wobei die aromatische Gruppe chloriert ist.
9. Verfahren nach Anspruch 8, wobei 2,4,5-Trichlorbenzolsulfonsäure verwendet wird.
10. Verfahren nach Anspruch 8, wobei Pentachlorphenol verwendet wird.
11. Verfahren nach Anspruch 8, wobei 2,6-Dichlorbenzoesäure verwendet wird.
12. Verfahren nach Anspruch 6, wobei p-Toluolsulfonsäure verwendet wird.
13. Verfahren nach einem der vorangehenden Ansprüche, wobei die Palladiumverbindung ein Palladiumsalz einer organischen Säure ist.
14. Verfahren nach einem der vorangehenden Ansprüche, das durchgeführt wird bei einer Temperatur im Bereich von 75°C bis 200°C.
15. Verfahren nach einem der vorangehenden Ansprüche, das durchgeführt wird bei einem Druck im Bereich von 10 bis 150 bar.

**Revendications**

1. Procédé de préparation d'isocyanates organiques, qui consiste à faire réagir un composé nitré aromatique avec du monoxyde de carbone, ce procédé étant mis en œuvre en présence d'un système catalytique préparé en combinant:
a) du palladium et/ou un composé du palladium,
b) un ligand de formule générale I

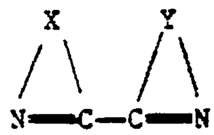

(I)

dans laquelle X et Y sont des groupes pontants identiques ou différents comportant chacun 3 ou 4 atomes de carbone dans le pont, dont au moins deux sont des atomes de carbone, ces groupes X et Y pouvant être liés l'un à l'autre, et

c) un ou plusieurs acides protoniques, à l'exception des acides halogénhydriques.

2. Procédé selon la revendication 1, dans lequel X et Y sont aussi liés l'un à l'autre au moyen d'un raccordement autre que celui formé par les atomes de carbone indiqués dans la formule I.

3. Procédé selon la revendication 2, dans lequel le ligand est la 1,10-phénanthroline ou un de ses dérivés.

4. Procédé selon la revendication 2, dans lequel le ligand est le 2,2'-bipyridyle ou un de ses dérivés.

5. Procédé selon l'une quelconque des revendications précédentes, dans lequel on emploie un ou plusieurs acides protoniques de formule générale II

RQ (II)

dans laquelle R représente un groupe aromatique éventuellement substitué et Q représente un groupe $SO_3H$, OH ou C(O)OH.

6. Procédé selon la revendication 5, dans lequel R représente un groupe phényle éventuellement substitué.

7. Procédé selon la revendication 5 ou 6, dans lequel le groupe aromatique est halogéné.

8. Procédé selon la revendication 7, dans lequel le groupe aromatique est choré.

9. Procédé selon la revendication 8, dans lequel on utilise l'acide 2,4,5-trichlorobenzènesulfonique.

10. Procédé selon la revendication 8, dans lequel on utilise le pentachlorophénol.

11. Procédé selon la revendication 8, dans lequel on utilise l'acide 2,6-dichlorobenzoïque.

12. Procédé selon la revendication 6, dans lequel on utilise l'acide p-toluènesulfonique.

13. Procédé selon l'une quelconque des revendications précédentes, dans lequel le composé de palladium est un sel de palladium d'un acide organique.

14. Procédé selon l'une quelconque des revendications précédentes, qui est mis en œuvre à une température dans l'intervalle de 75°C à 200°C.

15. Procédé selon l'une quelconque des revendications précédentes, qui est mis en œuvre sous une pression dans l'intervalle de 10 à 150 bars.